(19) Europäisches Patentamt
European Patent Office
Office européen des brevets

(11) Numéro de publication: **0 151 062**
**A2**

(12) **DEMANDE DE BREVET EUROPEEN**

(21) Numéro de dépôt: **85400086.6**

(22) Date de dépôt: **18.01.85**

(51) Int. Cl.⁴: **C 07 D 409/12**, C 07 D 401/12, C 07 D 407/12, C 07 D 241/20, C 07 D 239/42, A 61 K 31/00

(30) Priorité: **18.01.84 FR 8400757**

(43) Date de publication de la demande: **07.08.85 Bulletin 85/32**

(84) Etats contractants désignés: **AT BE CH DE FR GB IT LI LU NL SE**

(71) Demandeur: **RIOM LABORATOIRES- C.E.R.M. "R.L.-CERM" - (S.A.), Route de Marsat B.P. 140, F-63203 Riom Cédex (FR)**

(72) Inventeur: **Combourieu, Michel, 6, rue du Mont Mouchet, F-15000 Aurillac (FR)**
Inventeur: **Simond, Jacques, Rue des Thuilets Mirefleurs, F-63730 Les-Martres-de-veyre (FR)**
Inventeur: **Monteil, André, Route de Prompsat Les Grosliers, F-63140 Chatel-Guyon (FR)**

(74) Mandataire: **Thérond, Gérard Raymond et al, RIOM LABORATOIRES - CERM - S.A. Route de Marsat B.P. 140, F-63203 Riom Cédex (FR)**

(54) **3-Alkoxy-2-(N-pyrrolidino)-N-pyrimidinyl ou N-pyrazinyl-propylamines, leur préparation et les compositions pharmaceutiques les contenant.**

(57) La présente invention a pour objet de nouveaux composés de formule générale

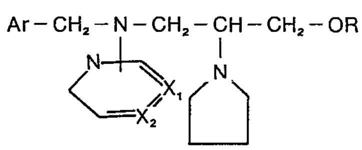

dans laquelle Ar représente un groupe thiényle, furyle, pyridyle ou phényle éventuellement substitué;
R représente un radical alkyle linéaire ou ramifié de 1 à 7 atomes de carbone; et l'un des deux substituants $X_1$ ou $X_2$ représente l'azote, l'autre étant un carbone, et leurs sels d'addition acide pharmaceutiquement acceptables, possédant d'intéressantes propriétés cardiovasculaires.

EP 0 151 062 A2

La présente invention a pour objet de nouvelles 3-alkoxy -2-(N-pyrrolidino)-N-pyrimidinyl ou N-pyrazinyl propylamines, des méthodes pour leur préparation et les compositions pharmaceutiques les contenant.

Ces composés répondent plus précisément à la formule générale suivante :

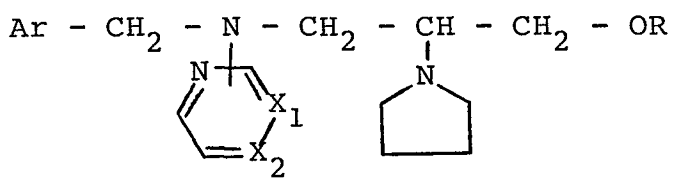

(I)

dans laquelle Ar représente un groupe thiényle, furyle, pyridyle ou phényle éventuellement substitué ; R représente un radical alkyle linéaire ou ramifié de 1 à 7 atomes de carbone ; et l'un des substituants $X_1$ ou $X_2$ représente l'azote, l'autre étant un carbone, et leurs sels d'addition acide pharmaceutiquement acceptables.

Les composés de l'invention possèdent d'intéressantes propriétés cardiovasculaires et ils exercent plus particulièrement d'importantes activités anti-angineuse, anti-hypertensive et anti-dysrythmique.

Les composés de formule I peuvent être préparés par des méthodes connues pour la préparation de composés analogues.

Les composés I peuvent être préparés en faisant réagir le composé de formule II

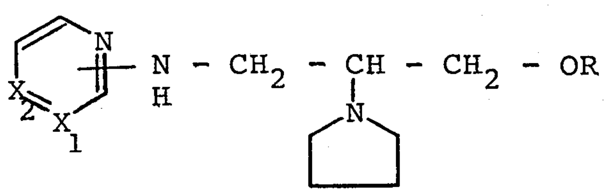

(II)

avec une amine de formule III

$$Ar\ CH_2 - Hal \qquad (III)$$

dans lesquels Ar, R, $X_1$ et $X_2$ ont les significations précédemment indiquées et Hal représente un halogène de préférence le chlore ou le brome.

Le composé de départ de formule II peut par exemple être préparé en faisant réagir un composé de formule IV

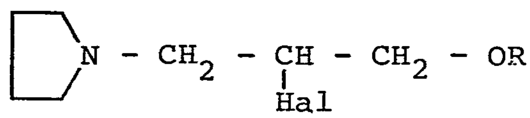 (IV)

dans laquelle Hal et R ont les significations précédemment indiquées avec une aminopyrimidine ou une aminopyrazine. Cette réaction se déroule de préférence en traitant d'abord l'aminopyrimidine ou l'aminopyrazine par un agent de métallation alcalin tel que l'hydrure ou l'amidure de sodium.

Une autre méthode de préparation des composés de formule I consiste à faire réagir un composé de formule IV avec un composé de formule V

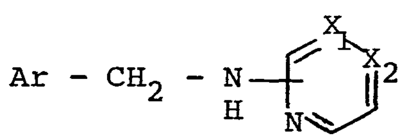 (V)

dans laquelle Ar, $X_1$ et $X_2$ ont les significations précédemment indiquées. De préférence, la réaction d'un composé IV avec un composé V est facilitée en effectuant d'abord une métallation du dernier composé au moyen d'un agent de métallation alcalin tel que l'hydrure de sodium ou l'amidure de sodium, potassium ou lithium.

Le composé de formule V peut être obtenu en faisant réagir l'aldéhyde ArCHO, dans laquelle Ar a la signification précédemment indiquée avec une aminopyrimidine ou une aminopyrazine pour donner une imine qui est ensuite réduite in situ pour donner l'amine secondaire désirée de formule V. Un agent de réduction approprié est par exemple le borohydrure de sodium.

Les composés de formule générale I et leurs sels d'addition acide se sont révélés posséder d'intéressantes propriétés anticalciques mises en évidence in vitro par les méthodes usuelles de pharmacologie moléculaire [VAN ROSSUM Arch. Int. Pharmacodyn. Ther. 143, 299-330, (1963)] .

In vivo, on a recherché l'activité antiangineuse en mesurant chez le chien anesthésié les paramètres hémodynamiques habituels (pourcentage de variation et durée d'action) :

- Fréquence cardiaque à l'aide d'électrodes à E C G sous-cutanées.
- Débit artériel coronaire à l'aide d'un débitmètre électromagnétique.
- Action antitachycardisante (inhibition des effets chronotropes positifs de l'isoprénaline).

Les composés de l'invention sont administrés par voie I.V. à la dose de 5 $mg.kg^{-1}$ et les résultats rapportés dans le tableau I ci-après.

TABLEAU I

| COMPOSE N° * | FREQUENCE CARDIAQUE Variation (%) | FREQUENCE CARDIAQUE Durée (min) | DEBIT CORONAIRE Variation (%) | DEBIT CORONAIRE Durée (min) | ACTION ANTI-TACHYCARDISANTE Variation (%) | ACTION ANTI-TACHYCARDISANTE Durée (min) |
|---|---|---|---|---|---|---|
| 1 | - 17 | 35 | + 252 | 30 | - 46 | 30 |
| 3 | - 16 | >45 | + 32 | 1 | - 70 | >45 |
| 4 | - 33 | >45 | + 150 | >45 | - 42 | 30 |
| 5 | - 18 | >45 | + 86 | 5 | - 50 | 30 |
| 6 | - 21 | 60 | + 196 | 45 | - 44 | 30 |
| 7 | - 5 | 2 | + 67 | 2 | - 58 | 5 |

* voir tableau II pour la structure chimique des composés testés.

Les résultats hémodynamiques enregistrés " in vivo " montrent que les composés de l'invention présentent des activités bradycardisantes et antitachycardisantes importantes et de longue durée. Les composés n$^{os}$ 1 et 6 sont ceux qui possèdent l'activité coronarodilatatrice la plus importante pendant une longue durée.

Les composés de l'invention se sont en outre révélés posséder une toxicité réduite : leur toxicité aiguë par voie orale chez la souris est généralement supérieure à 500 $mg.kg^{-1}$.
Cet ensemble de propriétés pharmacologiques permet donc d'envisager l'application des composés de l'invention en thérapeutique en tant que médicament pour le traitement des troubles cardiovasculaires tels que l'angine de poitrine, l'hypertension ou les troubles du rythme.

Associés aux excipients pharmaceutiques habituels, ils pourront être administrés par voie orale ou intraveineuse, à des doses quotidiennes comprises entre 1 et 15 mg par kg de poids corporel. En thérapeutique humaine, on peut utiliser une dose journalière comprise entre 40 et 1200 mg, de préférence entre 100 et 800 mg.

Mélangés avec des excipients convenables, les composés I, ou un de leurs sels, peuvent être comprimés sous forme de prise unitaire tels que comprimés, pilules etc... ou peuvent être mis en gélules. Au moyen de liquides convenables, les composés peuvent aussi être utilisés en préparations injectables ou orales sous forme de solutions, suspensions ou émulsions.

Les composés de formule I possèdent un carbone asymétrique de sorte qu'il est possible d'obtenir un mélange racémique de I et les énantiomères optiques séparés. Le mélange racémique et les énantiomères optiques séparés appartiennent à la fois aux composés de l'invention.

Les énantiomères optiques peuvent être séparés de manière habituelle par résolution du mélange racémique, ou directement en partant de matières premières optiquement actives.

L'invention concerne aussi les sels d'addition acide des composés de formule I pharmaceutiquement acceptables. Ces sels sont obtenus de manière habituelle en combinant la base libre I avec des acides inorganique ou organique, tels que les acides chlorhydrique, fumarique, maléique, citrique ou succinique, ces acides n'étant mentionnés qu'à titre illustratif mais sans impliquer de limitation.

Dans la définition de Ar, un groupe phényle éventuellement substitué comprend un groupe phényle non substitué et un groupe phényle substitué par exemple par un halogène, un radical hydroxy, un groupe alkoxy ou alkyle de 1 à 4 atomes de carbone. Le radical paraméthoxy est le radical préféré.

Le groupe 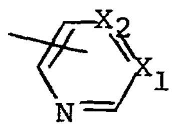 comprend les radicaux pyrimidinyle et pyrazinyle, dont les radicaux 2-pyrimidinyle et pyrazinyle sont les radicaux préférés.

Le groupe alkyle dans la définition de R est un groupe alkyle linéaire ou ramifié de 1 à 7 atomes de carbone tels que les radicaux méthyle, éthyle, propyle, butyle, isobutyle, pentyle, isopentyle et hexyle.
Dans la définition de R, le radical isobutoxy est le radical préféré.

Les composés préférés selon l'invention sont les composés de formule I et leurs sels d'addition acide dans lesquels, seuls ou en combinaison,
R représente le radical isobutyle,
le groupe 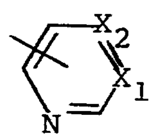 représente un radical 2-pyrimidinyle ou pyrazinyle et Ar représente un radical 2-furyle, 2-thiényle, phényle, p-méthoxy phényle ou 2-pyridyle.

Parmi les composés préférés ci-dessus de formule I, on retiendra en particulier les composés de formule I et leurs sels d'addition acide dans lesquels le groupe aromatique contenant deux atomes d'azote est le radical 2-pyrimidinyle et Ar représente soit le radical 2-furyle, soit le radical phényle, soit le radical p-méthoxy-phényle.

## EXEMPLE 1

3-isobutoxy-2-(N-pyrrolidino)-N-(2-pyrazinyl) N-(2-thienyl) méthyl propylamine

Dans une première étape, on a introduit dans un tricol de 2 litres sous agitation magnétique chauffante 28,5 g (0,3 M) de 2-amino pyrazine dans 400 ml de toluène, puis 7,5 g (0,25 M) d'hydrure de sodium à 80 %. On a porté le mélange à reflux pendant 1/2 heure, laissé revenir à température ambiante, introduit en une fois 65,9 g (0,3 M) de 2-chloro-1-isobutoxy-3-pyrrolidino propane et porté à reflux pendant 18 heures. Après avoir laissé le milieu réactionnel revenir à température ambiante, on a lavé à l'eau, séché sur sulfate de magnésium, filtré, évaporé le solvant à sec, puis distillé le résidu sous pression réduite.
On a ainsi obtenu 38 g de 3-(2-pyrazinyl) amino-2 pyrrolidino-1-isobutoxy propane ayant pour point d'ébullition $E_{0,05}$ = 160-164°C.

Dans une deuxième étape, on a introduit dans un tricol de 500 ml 16,2 g (0,058 M) de l'amine précédente dans 250 ml de toluène, puis 2,3 g (0,078 M) d'hydrure de sodium à 80 %, porté 2 heures à reflux, laissé revenir à température ambiante puis ajouté 8,5 g (0,064 M) de 2-chloro méthyl thiophène, puis porté le mélange à

reflux pendant 2 heures. Après avoir laissé le mélange revenir à température ambiante, on a détruit l'excès de NaH avec un peu d'eau saturée en NaCl puis lavé à l'eau, séché sur sulfate de magnésium, filtré, évaporé le solvant et distillé le résidu obtenu sous pression réduite.
On a ainsi obtenu 7,7 g de produit du titre ayant pour point d'ébullition $E_{0,05}$ = 190-192°C.

EXEMPLE 2

3-isobutoxy-2-(N-pyrrolidino)-N-(2-pyrazinyl) N-(4-methoxy benzyl) propylamine

Dans un premier stade, on a introduit dans un ballon 300 ml de toluène, 17,5 g (0,184 M) de 2-amino pyrazine et 31,4 g (0,184 M) d'aldéhyde anisique, puis on a porté le mélange au reflux azéotropique. Après avoir décanté 3 ml d'eau on a évaporé le toluène, repris par l'oxyde d'isopropyle, filtré et séché sous vide à 30°C le précipité obtenu. On a ainsi obtenu 26,4 g d'imine qu'on a dissous dans 250 ml de méthanol sous agitation magnétique au bain d'eau froide ; on a alors introduit par petites quantités 5,6 g de $NaBH_4$, puis laissé le mélange sous agitation 2 heures à température ambiante. Après avoir évaporé le méthanol, on a repris le mélange réactionnel par 300 ml d'éther, lavé à l'eau, séché la phase organique sur $Na_2SO_4$, filtré, évaporé l'éther et repris le résidu par 100 ml d'oxyde d'isopropyle. Après avoir filtré et recristallisé dans l'éthanol absolu, on a obtenu 16,7 g de 2-(4-méthoxy phényl) aminométhyl pyrazine ayant pour point de fusion F = 105,3°C.

Dans un deuxième stade, on a dissous, sous agitation magnétique chauffante 13,9 g (0,0645 M) de l'amine précédente dans 250 ml de toluène, puis on a introduit 2,6 g (0,087 M) de NaH à 80 % et porté le mélange 1 h 30 à reflux. Après avoir laissé revenir à température ambiante, on a introduit 14,2 g (0,0645 M) de 2-chloro-1-isobutoxy-3-pyrrolidino propane, puis porté le mélange 6 heures à reflux. Après avoir laissé revenir à température ambiante, on a hydrolysé avec un peu d'eau saturée en NaCl, lavé à l'eau, décanté, séché la phase organique sur $MgSO_4$ filtré, évaporé le toluène, distillé l'huile résiduelle en gardant la fraction passant à 200-202°C sous 0,05 mm de mercure. Après avoir dissous cette fraction dans 100 ml d'acétone, on a fait barboter un courant d'acide chlorhydrique anhydre jusqu'à pH 1. Après séparation du précipité et purification par les moyens habituels, on a obtenu 8,5 g de composé du titre sous forme de dichlorhydrate ayant pour point de fusion F = 146,8°C.

## EXEMPLE 3

### 3-isobutoxy-2-(N-pyrrolidino)-N-(2-pyrimidinyl)-N-benzyl propylamine

Dans une première étape, on a introduit dans un tricol de 2 litres sous agitation magnétique 19 g (0,20 M) de 2-amino pyrimidine dans 400 ml de toluène, puis 7,5 g (0,25 M) d'hydrure de sodium à 80 %, puis porté le mélange à reflux pendant 1/2 heure. Après avoir laissé le mélange revenir à température ambiante, on a introduit en une seule fois 46,14 g (0,21 M) de 2-chloro-1-isobutoxy-3 pyrrolidino propane et porté à reflux pendant 3 heures.

Après avoir laissé le milieu réactionnel revenir à température ambiante, on a lavé à l'eau, séché sur sulfate de magnésium, filtré, évaporé le solvant à sec puis distillé le résidu sous pression réduite.
On a ainsi obtenu 22 g de 3-isobutoxy-2-(N-pyrrolidino)-N-(2-pyrimidinyl) propylamine ayant pour point d'ébullition $E_{0,01}$ = 152-156°C et pour point de fusion F = 40,7°C.

Dans une deuxième étape, on a introduit 14 g (0,046 M) de l'amine précédente dans un ballon de 500 ml contenant 250 ml de toluène, puis 2,2 g (0,073 M) d'hydrure de sodium et porté le mélange à reflux pendant 3 heures 1/2. Après avoir laissé revenir à température ambiante, on a ajouté 7 g (0,055 M) de chlorure de benzyle et porté le mélange à reflux pendant 1 heure.
Après avoir laissé revenir à température ambiante, on a hydrolysé l'excès de NaH, lavé à l'eau, séché sur $MgSO_4$, filtré, évaporé le solvant et distillé l'huile résiduelle sous pression réduite.
On a ainsi obtenu 5 g de produit du titre ayant pour point d'ébullition $E_{0,05}$ = 167,5°C.

## EXEMPLE 4

Les composés suivants — résumés dans le tableau II — ont été obtenus de manière analogue en utilisant la méthode de l'exemple 1 ou celle de l'exemple 2.
Pour être complet, le tableau II inclut les composés préparés dans les exemples précédents.
Tous les composés du tableau II ont la formule générale II dans laquelle R représente le radical isobutyle.

## TABLEAU II

| COMPOSE N° | Ar | 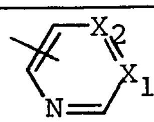 | SEL POINT DE FUSION ou D'EBULLITION | PREPARE SELON |
|---|---|---|---|---|
| 1 | 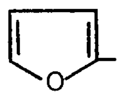 | 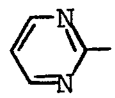 | Base $E_{0,05}$ = 205°C<br>Fumarate F = 98,2°C | Ex. 1 |
| 2 (Ex.1) | 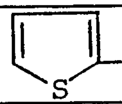 | 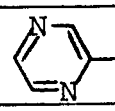 | Base $E_{0,05}$ = 191°C | Ex. 1 |
| 3 | 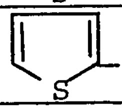 | 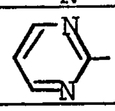 | Base $E_{0,05}$ = 171°C | Ex. 1 |
| 4 | 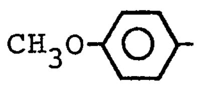 | 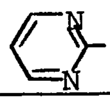 | Base $E_{0,05}$ = 220°C | Ex. 2 |
| 5 (Ex.2) | 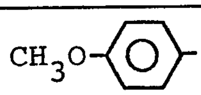 | 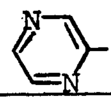 | 2 HCl F = 146,8°C | Ex. 2 |
| 6 (Ex.3) | 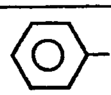 | 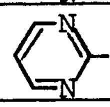 | Base $E_{0,05}$=167,5°C | Ex. 3 |
| 7 | 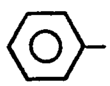 | 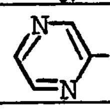 | Base $E_{0,05}$ = 183°C | Ex. 1 |
| 8 | 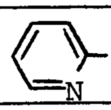 | 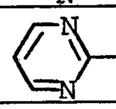 | Base $E_{0,05}$ = 169°C | Ex. 1 |

# REVENDICATIONS

1. Composés de formule générale

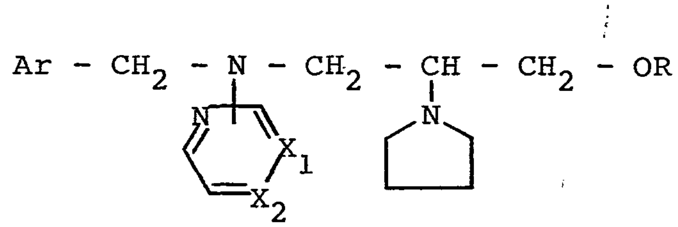

(I)

dans laquelle Ar représente un groupe thiényle, furyle, pyridyle ou phényle éventuellement substitué; R représente un radical alkyle linéaire ou ramifié de 1 à 7 atomes de carbone ; et l'un des deux substituants $X_1$ ou $X_2$ représente l'azote, l'autre étant un carbone, et leurs sels d'addition acide pharmaceutiquement acceptables.

2. Composé selon la revendication 1 caractérisé en ce que R représente le radical isobutyle.

3. Composé selon la revendication 1 caractérisé en ce que le groupe 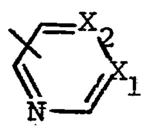 représente le radical pyrazinyle.

4. Composé selon la revendication 1 caractérisé en ce que le groupe 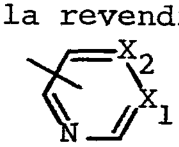 représente le radical 2-pyrimidinyle.

5. Composé selon la revendication 1 caractérisé en ce que Ar représente un radical 2-furyle, 2-thiényle, phényle, p.méthoxyphényle ou 2-pyridyle.

6. Composé selon la revendication 4 caractérisé en ce que R représente le radical isobutyle et Ar désigne un radical 2-furyle, phényle ou p-méthoxy.

7. Composé selon la revendication 6 caractérisé en ce que Ar représente le radical 2-furyle.

8. Composition pharmaceutique caractérisée en ce qu'elle contient à titre de principe actif au moins un des composés selon la revendication 1, en association avec un ou plusieurs excipients convenables.

9. Procédé pour la préparation de composés selon la revendication 1 caractérisé en ce qu'on fait réagir un composé de formule II (voir description) avec une amine de formule III (voir description) et qu'on peut ensuite transformer le composé de formule I obtenu en un sel d'addition acide pharmaceutiquement acceptable.

10. Procédé pour la préparation de composés selon la revendication 1 caractérisé en ce qu'on fait réagir un composé de fomule IV (voir description) avec une amine de formule V (voir description) et qu'on peut ensuite transformer le composé de formule I obtenu en un sel d'addition acide pharmaceutiquement acceptable.